# EUROPEAN PATENT APPLICATION

(11) **EP 3 301 345 A1**
(43) Date of publication of application: **04.04.2018**
(21) Application number: 16191140.9
(22) Date of filing: 28.09.2016
(51) Int. Cl.: F17C 13/08

(54) **CYLINDER HOLDER, SUPPLY SYSTEM AND SUPPLY METHOD FOR THERAPEUTIC OXYGEN**

(71) Applicant: Linde Aktiengesellschaft, 80331 München (DE)
(72) Inventor: FRANZ, Helmut, 82319 Starnberg (DE); DROTT, Kenth, 82069 Hohenschäftlarn (DE)
(74) Representative: m patent group

(57) **Abstract**

The present invention relates to a cylinder holder (10) for a cylinder (20) for pressurized storage of therapeutic oxygen, the cylinder holder (10) comprising a receptacle (11) arranged to hold the cylinder (20) in a holding position, characterized in that the cylinder holder (10) comprises at one or more than one cylinder switch (12) adapted to be actuated by the cylinder (20) when received in the receptacle (11) in the holding position. A corresponding supply system (100) for therapeutic oxygen and a corresponding supply method are also part of the present invention.

## Description

The present invention relates to a cylinder holder, to a supply system and to a supply method for therapeutic oxygen according to the pre-characterizing parts of the independent claims.

### Prior Art

Advances in therapeutic uses of oxygen have resulted in its increased application for a number of clinical conditions, e.g. hypoxia and hypoxemia, respiratory depression, chronic obstructive pulmonary disease, pneumoconiosis, pneumonia, heart attack and pulmonary embolism, respiratory distress syndrome in new-borns, cluster headaches, respiratory burns and carbon monoxide poisoning. Classically, oxygen is used during surgery to maintain tissue oxygenation under anaesthesia, in resuscitation and for mechanical ventilation of the lung for the treatment of respiratory depression.

Oxygen for use in hospitals may be delivered in liquid state by tank trucks and distributed to the points of use, e.g. wards or operation theatres, from a central distribution point via pipe systems and wall outlets. However, oxygen cylinders are also in common use in hospitals, especially if smaller quantities of oxygen are required and/or if for other reasons providing a central oxygen supply system is not recommended, feasible and/or necessary. A further important advantage of oxygen cylinders is their mobility, and therefore their applicability in patient transportation under uninterrupted oxygen supply.

While the present application mainly describes the use of oxygen in medical contexts, the invention is useful in any field in which oxygen is provided in a gas cylinder, e.g. for industrial applications such as welding or heating or for laboratory applications where oxygen is used in chemical reactions.

In clinical contexts, there may be a problem to evaluate the amount of cylinders needed, e.g. in a ward in a hospital. For example, in a typical ward, a certain number, e.g. eight, oxygen cylinders are placed in wall cylinder holders. When oxygen is needed for e.g. the transport of a patient, a cylinder is dismounted from the wall cylinder holder and typically hung to the bed of the patient.

If proper documentation and/or a notification to a facility responsible for the distribution of oxygen cylinders within a hospital is omitted in such situations, there might be the consequence that a ward is no more equipped with a significant number of oxygen cylinders, e.g. for further patient transport. Especially in emergency situations, this might have severe consequences with regard to patient safety.

It is an object of the present invention to provide for an improved supply for therapeutic oxygen which provides increased reliability and manageability.

### Disclosure of the invention

This object is solved by a cylinder holder, a supply system and a supply method for therapeutic oxygen including the features of the independent claims. Preferred embodiments of the invention and further features are subject of the dependent claims and of the description that follows.

### Advantages of the invention

According to the present invention, a cylinder holder for a cylinder for pressurized storage of therapeutic oxygen is provided. The cylinder holder comprises a receptacle arranged to hold the cylinder in a holding position. According to the present invention, the cylinder holder comprises one or more than one cylinder switch adapted to be actuated by the cylinder when received in the receptacle in the holding position.

Cylinder holders for gas cylinders are generally known from the prior art. Typically, a cylinder holder is adapted to receive and hold a gas cylinder, especially a pressurized gas cylinder, of only a certain size. Typically, a cylinder holder comprises a frame or another holding structure at least partially surrounding the cylinder to be held by the cylinder holder. Furthermore, typically, in corresponding cylinder holders, securing means such as chains or belts are provided which especially serve to prevent tilting over of the cylinder which could result in damage and dangerous situations, especially in the case of pressurized gas cylinders. For an example of a cylinder holder, reference is made e.g. to WO 2010/109206 A2.

If, in the following, reference made is to "a" cylinder holder in singular, the corresponding explanations equally apply to an arrangement of cylinder holders or a larger structure comprising the cylinder holder or several receptacles. Such a larger structure is also referred to a cylinder holder but, in contrast to a cylinder holder for only one gas cylinder, comprises more than one receptacle to receive several cylinders.

The basic idea of the present invention is to have, in a corresponding cylinder holder, a button, actuator, contact or the like, herein generally referred to as a "switch", which is pressed down or otherwise operated or actuated when a cylinder is present in the receptacle of the cylinder holder and e.g. held in place by securing means as described. If a corresponding switch is thus operated by a corresponding cylinder, this indicates that a cylinder is present in the holder and is ready to be used for the purposes as mentioned above, e.g. for transporting a patient. If a cylinder holder according to the present invention e.g. comprises eight receptacles for receiving cylinders, and if each of these receptacles comprises one or more switches from which seven are operated, one can conclude that e.g. seven cylinders are present because, then, seven corresponding switching signals or signals indicating that a corresponding switch is operated, are present. Such signals, as later explained, may especially be transmitted to a central station of a supply system for therapeutic oxygen, where such a signal may be evaluated as also explained below.

If, in the context of the present application, reference made is to "switch", this term may include a mechanical switches or other switches as generally known in the art. For example, the present invention also may include photoelectric switches like light barriers which comprise a light emitting unit and a light detecting unit. If, in a corresponding light barrier arrangement, a cylinder is placed within the light path, the light path is interrupted and the light receiving unit may not receive light from the light emitting unit. On this basis, one may conclude that a cylinder is present and held in the receptacle. Other types of photoelectric switches may also be provided, e.g. a simple light sensor switch are shaded with a corresponding cylinder is placed in the receptacle and thus covers the photo sensor. As such photoelectric sensors may be erroneously operated, e.g. if a user covers the light path and/or a corresponding light detection unit with another item than a cylinder, special care must be taken to avoid false-positive detections. For example, a redundant further switch may be provided or the light path of the photoelectric switch may be placed in a position which is not easily or regularly accessible by an operator. An example may be a key/keyhole arrangement wherein the light path may be arranged in a recession with which an, e.g. geometrically coded, protrusion of the cylinder is adapted to mate.

A corresponding switch may also be embodied as a switch based on detecting a conductivity and/or resistivity and/or inductivity signal, which only is present, e.g. between to contacts of a corresponding switch, as if a metallic element, like an oxygen cylinder, is present in the receptacle. Further types of switches are also contemplated with the context of the present invention. Corresponding switches may also be configured to be operated only if a certain switching force is exerted upon the switch, i.e. upon a mechanical switch, such a force corresponding to a filled cylinder and not an empty cylinder. Using a corresponding switch, a differentiation may be made between a filled cylinder, an empty cylinder and/or another item not being a cylinder, which is e.g. misusedly placed in the receptacle. In all cases, the key/keyhole arrangement as described may be used.

In this context, it should also be noted that, for example, also a scale can be provided in the cylinder holder, the scale being adapted to establish the weight of the cylinder and therefore the content. Using a corresponding scale or balance, a differentiation between filled, and therefore ready to use, and empty cylinders may be made. This further contributes to patient safety and reliability of such a cylinder holder and a corresponding supply system.

Overall, using a corresponding cylinder holder, the cylinder logistics, e.g. in a hospital, may be improved and possible confusion about cylinder whereabouts and an the actual oxygen consumption may be reduced. By knowing how many of the cylinder holders or how many receptacles within one cylinder holder are empty, and for how long, the oxygen consumption in a ward can be estimated and the refilling of full cylinders can be organized and scheduled according to the demands.

According to a particularly preferred embodiment of the present invention, a corresponding cylinder holder is not a standalone unit but such a cylinder holder comprises a transmitter which is adapted to transmit a signal indicative of a switching state and/or a switching operation of the one or more than one cylinder switch. The status of filling of the cylinder holder or of one or more receptacles within the cylinder holder may therefore communicate per central unit and evaluated therein. This further improves, in contrast to a standalone cylinder holder, the cylinder logistics, especially in larger medical units. On the basis of a corresponding signal, a priorisation and/or a distribution schedule for cylinders may be established. Such an approach is particularly time-saving, because checking of all cylinder holders within a hospital or every ward within a hospital can be omitted.

According to a particularly preferred embodiment, a cylinder holder according to the present invention comprises at least two cylinder switches, wherein the transmitter is adapted to transmit the signal only when all of the at least two cylinder switches are in a defined state and/or if they are all operated within a predefined time frame.

By, for example, providing two cylinder switches per cylinder holder or cylinder receptacle, erroneous signals can be reduced. If, for example, one of the cylinder switches is unintentionally switched by an operating person or a visitor in the ward, for example, this does not yet result in a signal to be transmitted by the transmitter. A signal is only transmitted if both switches are operated, which preferentially is only be the case if the cylinder contacts or operates over its whole length both switches. If a cylinder is placed in such an arrangement, it is expected that both switches are operated more or less coincidentally. Therefore, switching operations of the two switches are expected within a certain time frame, other than a switching operation which is performed due to misuse of the cylinder holder. This further increases reliability and safety of a corresponding arrangement.

Other arrangements are also contemplated. For example, two different cylinder switches can be provided, the cylinder switches operating on different switching principles, e.g. a mechanical switch and at least one of a photoelectric switch, a resistive switch, a inductive switch etc. In such an arrangement, the switching signal from one switch can be verified by the switching signal of another switch operating according to an orthogonal operating principle. The likelihood of a false-positive cylinder detection can thus be reduced.

According to a particularly preferred embodiment of the present invention, the transmitter may also be adapted to transmit a signal indicative of the number of the cylinder switches which are in a defined state and/or which are operated within a predefined time frame. These cylinder switches may, like before, also be provided within one receptacle or within one cylinder holder for only one cylinder.

Using a corresponding arrangement, according to a particularly preferred embodiment, a size of a cylinder placed in the receptacle may also be determined. For example, different cylinders for oxygen may comprise an identical diameter but a different height. Therefore, if cylinder switches are arranged in different heights, e.g. at the back of a cylinder holder, different numbers of them may be operated depending on the height, and, therefore, the volume of the cylinder. A corresponding cylinder holder may thus provide, further to a mere detection whether a cylinder is present of not, also an indication of the size of the cylinder.

To this purpose, a cylinder holder according to a particularly preferred embodiment of the present invention comprises cylinder switches which are arranged in a way that depending on a size of the cylinder which is held in the holding position, a different number of the cylinder switches is operated. Again, also in this embodiment, different switching principles for mutual confirmation may be provided.

A cylinder holder according to a further preferred embodiment of the invention further comprises one or more user switches, wherein the transmitter is adapted to transmit a signal indicative of the state of the one or more user switches. A corresponding user switch may for example be used to indicate that a cylinder is still present but empty. If such a determination is not made by the cylinder holder itself, e.g. via a scale or balance as explained above or a corresponding switching force, a central unit of a corresponding oxygen supply system may be informed of empty cylinders which are to be replaced. This further provides an increased patient safety and improves the manageability of a corresponding oxygen supply system.

While, as explained above, a cylinder holder or a receptacle of such cylinder holder may allow placing cylinders of different sizes within the receptacle, a corresponding cylinder holder may also be adapted to allow only for holding a cylinder of a predetermined size in the holding position. In such an embodiment, a size determination as explained above can be omitted but a central unit used in a corresponding system may still rely on a signal from a corresponding cylinder holder because only a one size of cylinders may be placed in a corresponding receptacle and, therefore, if a signal indicating the presence of a cylinder in such a receptacle is present, also the size of the corresponding cylinder is known.

According to a particularly preferred embodiment of the present invention, a cylinder holder is further adapted to transmit a signal indicative of a malfunction and/or a maloperation and/or a power failure of the cylinder holder. This further improves the reliability of corresponding system. According to another embodiment of the present invention, absence of any signal, e.g. a trigger signal in the kind of a "dead man signal" indicating that the cylinder holder is operating or responsive, may be present, ensuring that a cylinder holder or holders are operable.

It will be understood, that a "signal" according to the present invention may include any type of signal as known in the art, including wire-based signals or wireless signals, analogue or digital signals, optical, electrical or acoustical signals. Particularly preferred signals are wireless signals, especially Bluetooth, Wifi or Zigbee signals or signals corresponding to other transmission protocols or principles.

The present invention also relates to a supply system for therapeutic oxygen, the supply system comprising at least one cylinder holder for a cylinder for pressurized storage of therapeutic oxygen, the at least one cylinder holder comprising at least one receptacle arranged to hold the cylinder in a holding position. According to the present invention, the cylinder holder is a cylinder holder as explained above and below in all its embodiments. For advantages and features of such a supply system, reference is therefore made to the explanations above.

According to a particularly preferred embodiment, as already partially mentioned above, such a supply system further comprises a central unit, the central unit being adapted to receive one or more than one signals of the transmitter of at least one cylinder holder. Such a signal may be the signal which is indicative of the switching state and/or the switching operation of the one or more than one cylinder switch, the signal indicative of the number of cylinder switches which are in a defined state and/or which are operated within a predefined time frame, the signal indicative of the state of the one or more user switches and/or the signal indicative of the malfunction and/or maloperation and/or the power failure of the cylinder holder as explained above.

The central unit may be adapted to receive and process any number of such signals of any number of cylinder holders. In this embodiment, the central unit becomes a hub in a corresponding supply system which centralizes the basic function of the oxygen supply system. The central unit may be connected to further units, e.g. computer nets or servers, or the central unit may be embodied as or as a part of a computer in an oxygen distribution facility. To be able to receive signals from the transmitters of the cylinder holders, the central unit may comprise a corresponding receiver adapted to receive a wireless or wired signal as explained above.

According to a particularly preferred embodiment, the central unit is further adapted to perform a grouping of the cylinder holders into two or more groups when a plurality of cylinder holders is present. Such groups may for example be predefined by a user of the system and may correspond to different operating units of a hospital or different wards wherein cylinder holders with corresponding cylinders are present. In this embodiment, the oxygen (cylinder) needs of each group, e.g. each ward, may be resolved and individually fulfilled. It is, in this embodiment, for example also possible to perform statistics on oxygen usage within the different groups. On the basis of corresponding usage data, oxygen supply may be tailored to the needs of different units or groups and may e.g. priorized for certain units.

In this context, it is particularly of advantage if the central unit is further adapted to calculate at least one characteristic of the two or more groups on the basis of the one or more than one signals received from the transmitter of at least one of the cylinder holders. Such a characteristic may, as mentioned, be for example an oxygen usage over time, a peak oxygen usage, a frequency of oxygen usage and/or a duration of oxygen usage. Having such data available, corresponding supply system may be fine-tuned to avoid unnecessary oxygen delivery and/or oxygen shortages.

The corresponding system may particularly be used in connection with a so called intelligent cylinder. In this embodiment, the supply system further comprises at least one cylinder adapted to transmit a signal indicative of at least one characteristic of the cylinder. Corresponding "intelligent" cylinders can communicate for example their content on the basis of a measurement as known in the art and further information via wireless signals (Bluetooth etc.). In e.g. a ward there may be a monitoring system, also referred to as a "hub", which receives the signals from the intelligent cylinders and, in a computer system, can allocate the specific cylinder to the respective cylinder holder. Purely mechanical cylinders can not be identified in such a monitoring system. For example, by evaluating the content of an intelligent cylinder this information may be considered as a representative value for all the cylinders present in a corresponding holder, even if all further cylinders are purely mechanical and are not adapted to transmit corresponding information. In this embodiment, existing systems can be smoothly upgraded without replacing all mechanical cylinders by "intelligent" ones at one point of time. To this purpose, the central unit may be further adapted to differentiate between cylinders which are adapted to transmit a signal indicative of at least one characteristic of the cylinder and cylinder switch are not adapted to transmit such a signal. For example, in this embodiment, thus in the supply system information may be provided at which positions how many intelligent cylinders and how many purely mechanical cylinders are present.

The present invention relates also to a supply method for therapeutic oxygen, which, according to the present invention, comprises at least one cylinder holder as explained above and/or that corresponding oxygen supply system is used. Also as to the advantages of such systems, reference is made to the explanations above.

The invention and further embodiments of the invention are explained with reference to the appended drawing.

### Short description of the drawings

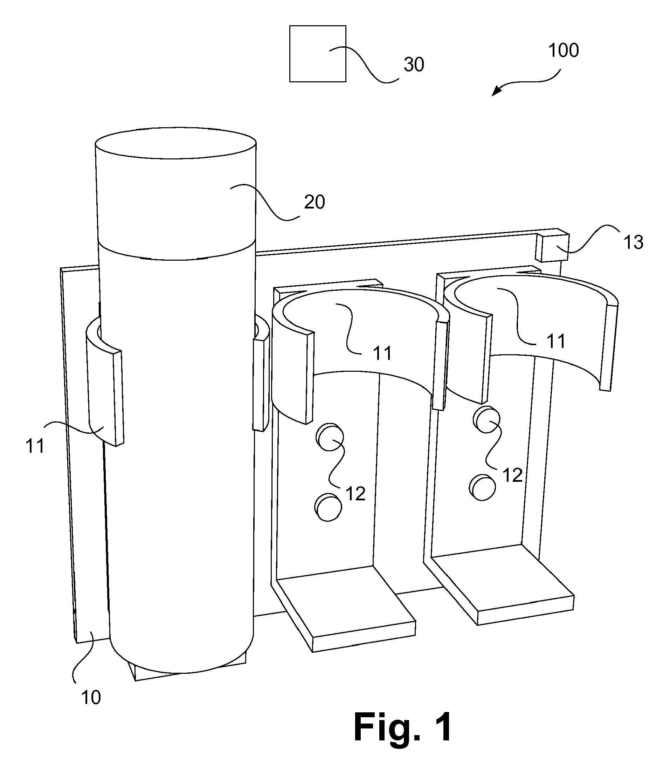

Figure 1 illustrates a supply system for therapeutic oxygen according to an embodiment of the present invention.

### Embodiment of the invention

In Figure 1, a supply system for therapeutic oxygen according to a particularly preferred embodiment of the present invention is illustrated and designated 100.

The system 100 comprises a cylinder holder 10 which, in the embodiment illustrated, comprises three receptacles 11 arranged to hold cylinders 20 for pressurized storage of therapeutic oxygen. The leftmost receptacle 11 is shown as being supplied with a corresponding cylinder 20, the two receptacles 11 on the right are shown empty. In the receptacles 11 shown on the right, cylinder switches 12 are visible (they are covered in the leftmost receptacle 11 by the cylinder 20).

The cylinder switches 12 are adapted to be actuated by the cylinder when the cylinder is received in the receptacles 11 in the holding position. In the embodiment shown, two cylinder switches 12 are present, allowing for an improved detection as explained above. A transmitter 13, which may be coupled to the cylinder switches 12, may be present, the transmitter 13 being adapted to transmit signals from the cylinder switches 12 and/or a user switch (not shown) as explained above.

The receptacles 11 of the cylinder holder 10 may be adapted to accommodate any size of cylinders, for example 2, 3, 5 and 10 litres. The cylinders may be secured to the receptacles 11 or the cylinder holder 10 via a securing mechanism (not shown).

The system 100 further comprises a central unit 30 which is adapted to receive and process the signal or signals for the cylinder holder 10 as explained above.

## Claims

1. A cylinder holder (10) for a cylinder (20) for pressurized storage of therapeutic oxygen, the cylinder holder (10) comprising a receptacle (11) arranged to hold the cylinder (20) in a holding position, **characterized in that** the cylinder holder (10) comprises one or more than one cylinder switch (12) adapted to be actuated by the cylinder (20) when received in the receptacle (11) in the holding position.

2. A cylinder holder (10) according to claim 1 further comprising a transmitter (13) adapted to transmit a signal indicative of a switch state and/or a switching operation of the one or more than one cylinder switch (12).

3. A cylinder holder (10) according to claim 2 comprising at least two cylinder switches (12) wherein the transmitter (13) is adapted to transmit the signal only when all of the at least two cylinder switches (12) are in a defined state and/or operated within a predefined time frame.

4. A cylinder holder (10) according to claim 2 or 3 wherein the transmitter (13) is adapted to transmit a signal indicative of the number of cylinder switches (12) which are in a defined state and/or operated within a predefined time frame.

5. A cylinder holder according to claim 4 wherein the cylinder switches (12) are arranged in a way that depending on a size of the cylinder (20) which is held in the holding position, a different number of the cylinder switches (12) is operated.

6. A cylinder holder (10) according to any one of claims 2 to 5 further comprising one or more user switches, wherein the transmitter (13) is adapted to transmit a signal indicative of the state of the one or more user switches.

7. A cylinder holder (10) according to any one of the preceding claims wherein the receptacle (11) is shaped to allow only for holding a cylinder (20) of a predetermined size in the holding position.

8. A cylinder holder (10) according to any one of the preceding claims wherein the transmitter (13) is further adapted to transmit a signal indicative of a malfunction and/or a maloperation and/or a power failure of the cylinder holder.

9. A supply system (100) for therapeutic oxygen, the supply system comprising at least one cylinder (10) holder for a cylinder (20) for pressurized storage of therapeutic oxygen, the cylinder holder (10) comprising a receptacle (11) arranged to hold the cylinder (20) in a holding position, **characterized in that** the cylinder holder (10) is a cylinder holder (10) as defined in any one of the preceding claims.

10. A supply system (100) according to claim 8 further comprising a central unit (30), the central unit (30) being adapted to receive one or more than one signals of the transmitter (13) of the at least one cylinder holder (10).

11. A supply system (100) according to claim 9 wherein the central unit (30) is further adapted to perform a grouping of the cylinder holders (10) into two or more groups when a plurality of cylinder holders (10) is present.

12. A supply system (100) according to claim 11 wherein the central unit (30) is further adapted to calculate at least one characteristic of the two or more groups on the basis of the one or more than one signals received from the transmitter (13) of at least one of the cylinder holders (20).

13. A supply system (100) according to any one of claims 9 to 12 further comprising at least one cylinder (20) adapted to transmit a signal indicative of at least one characteristic of the cylinder (20).

14. A supply system (100) according to claim 13 wherein the central unit (30) is further adapted to differentiate between cylinders (20) which are adapted to transmit a signal indicative of at least one characteristic of the cylinder (20) and cylinders which are not adapted to transmit such a signal.

15. A supply method for therapeutic oxygen, **characterized in that** at least one cylinder holder (10) according to any one of claims 1 to 8 and/or a supply system according to any one of claims 1 to 14 is used.
